# EUROPEAN PATENT SPECIFICATION

(11) **EP 1 103 270 B1**
(45) Date of publication and mention of the grant of the patent: **16.04.2003**
(21) Application number: 00931296.8
(22) Date of filing: 02.06.2000
(51) Int. Cl.: A61K 38/22, A61P 37/02

(54) **TREATMENT OF AUTOIMMUNE DISEASES IN MAMMALS**
BEHANDLUNG AUTOIMMUNERKRANKUNGEN IN SÄUGERN
TRAITEMENT DE MALADIES AUTO-IMMUNES CHEZ DES MAMMIFERES

(30) Priority: 04.06.1999 ES 9901235
(43) Date of publication of application: 30.05.2001
(73) Proprietor: UNIVERSIDAD COMPLUTENSE DE MADRID, E-28040 Madrid (ES)
(72) Inventor: PEREZ GOMARIZ, Rosa, Facultas de Ciencias Biolog., E-28040 Madrid (ES); LECETA MARTINEZ, Javier, Faculta de Ciencias Bio., E-28040 Madrid (ES); DELGADO MORA, Mario, Facultad de Ciencias Bio., E-28040 Madrid (ES); MARTINEZ MORA, Carmen, Facultad Ciencias Bio., E-28040 Madrid (ES)
(74) Representative: Carpintero Lopez, Francisco
(86) International application number: ES0000197
(87) International publication number: WO00074708

(56) References cited:
- WO-A1-99/53944
- GANEA D.: 'Regulatory effects of vasoactive intestinal peptide on cytokine production in central and peripheral lymphoid organs' ADVANCES IN NEUROIMMUNOLOGY vol. 6, no. 1, 1996, pages 61 - 74, XP002940636

## Description

### STATE OF THE ART

Endotoxic shock is still the main cause of death in hospitals. The strategies for combating the effects of endotoxic shock focus on counteracting the bacterial agents responsible for the effect, on restoring the haemodynamic parameters, preventing cellular activation and on modifying the action of the defence mechanisms (Boyd O; Current Opinion in Anaesthesiology 1996, 9:98).

It is currently accepted that the inflammatory response to bacterial products directly contributes to the development of endotoxic shock (Parillo JE; New England Journal of Medicine 1993, 328:1471). The toxic bacterial products and those released during tissue damage activate the defence mechanisms, implicating cells such as neutrophils, monocytes, macrophages and endothelial cells, and mediators such as cytokines, platelet activation factor, metabolites of arachidonic acid and nitric oxide, causing haemodynamic changes and organics harmful to the host (Moldawer LL; Critical Care Medicine 1994, 22:3). Many cytokines have been proposed as markers of the seriousness of the development of septic shock. The circulating levels of TNFα, IL-1, IL-6 and IL-8 have been correlated with the probability of overcoming a septic episode. TNFα and IL-1 administered to humans or to experimental animals reproduce many of the haemodynamic manifestations of septic shock (Tracey KJ and co-workers; Science 1986, 234:470) and its inhibition has been assayed by means of injection of antagonist receptors and neutralising monoclonal antibodies with different results (Fisher CJ and co-workers; Critical Care Medicine 1994, 22:12). Among the immunological markers, the levels of circulating IL-6 are the best indicators of the seriousness of the sepsis and of the possibilities of recovering from the episode (Liaw YS and co-workers.; Journal of the Formosan Medical Association 1997, 96:685).

Despite the advance in the knowledge of the mechanisms and of the technological and pharmacological progress there are still few results concerning an improvement in the data for death, which translate into a figure of around 200,000 per year in the United States and Europe (Vicent J-L and Chamlou R; Current Opinion in Anaesthesiology 1996, 9:146).

Inflammatory processes are a vital process for the survival of all complex organisms. Inflammation is a natural defence process of the organism against foreign agents. The accumulation and activation of leukocytes in places where the aggression takes place is a central occurrence in all inflammatory processes (Schaal TJ and Bacon KB; Current Opinion in Immunology 1994, 6:865). An insufficient inflammatory response may compromise the survival of the organism, but an excessive response, which may be due to failures in the mechanisms of deactivation of the process due to different causes, can lead to an inflammatory or autoimmune disease (Sacca R and co-workers; Current Opinion in Immunology 1997, 9:851). These diseases are an important cause of morbidity and mortality in mammals due to tissue damage associated with said processes.

Macrophages play a key role in the regulation of immune and inflammatory responses. The execution of these activities is mediated by a whole series of complex processes in which, among others, many products of macrophage origin intervene. As a response to the antigens, and according to their origin, the macrophages secrete pro-inflammatory cytokines and oxidising agents, such as TNFα, IL-6, IL-1β, IL-12 and nitric oxide (Laskin DL and co-workers; Annual Review of Pharmacology and Toxicology 1995, 35:655). TNFα and IL-6 are, among others, two factors that contribute to physiopathological changes associated with several states of chronic or acute inflammation. The macrophages, in addition, participate in the initiation, maintenance and control of immune responses, acting as potent antigen presenters, providing the T-lymphocytes with a double activation signal: the antigen-molecule complex of the main histocompatibility complex (MHC) and a co-stimulatory signal mediated by molecules of the B7 family (Lenschow DJ and co-workers; Annual Review of Immunology 1996, 14:233). The B7 molecules comprise two isoforms, B7.1 and B7.2, both of which are implicated in the stimulation of two different types of T helper cells (Th), Th1 and Th2, and both of these produces a set of different cytokines (Kuchroo VK and co-workers; Cell 1995, 80:707).

Activation of Th1 cells implies the production of IFNγ and IL-12, among other factors, and is associated with the production of antibodies of the IgG2a isotype and it is manifest as a reaction of delayed inflammatory type. The activation of Th2 cells implies the production of IL-4, IL-5 and IL-10, among other factors, is associated with the secretion of antibodies of the isotype IgG1, inhibits the delayed inflammatory response and is manifest as a humoral response (Constant SL y Bottomly K; Annual Review of Immunology 1997, 15:297). The factors that determine the differentiation of one or an other type of response are mainly the characteristics of the antigen presenting cells and the cytokines present in the microenvironment in which the response takes place: IL-12 determines the differentiation of Th1 cells while IL-4 does so with Th2. When both are present, the IL-4 effect predominates (O'Garra AO; Immunity 1998, 8:275). Numerous cases of inflammatory and autoimmune diseases are due to the activation of an inappropriate type of Th cell.

The vasoactive intestinal peptide (VIP) is a basic peptide containing 28 amino acids units whose sequence is (Mutt V and Said SI; European Biochemistry 1974, 42:581):

His-Ser-Asp-Ala-Val-Phe-Thr-Asp-Asn-Tyr-Thr-Arg-Leu-Arg-Lys-Gln-Met-Ala-Val-
Lys-Lys-Tyr-Leu-Asn-Ser-Ile-Leu-Asn-NH2

It was isolated firstly from the small intestine of pig and later was identified in the brain and in the nerve endings of the peripheral system. It was established that it was a neuropeptide with neuromodulating properties (Fahrenkrug J; Pharmacology and Toxicology 1993, 72:354). It takes its name from its peripheral vasodilatory properties. VIP has also been identified in mast cells of rat and in granulomas (Cutz E. and co-worders; Nature 1978, 275:661). Immunochemical studies carried out in histological sections of the thymus, spleen and lymphatic ganglia of rat have identified immunoreactive VIP in lymphocytes of these organs (Gomariz RP and co-workders; Annals of the New York Academy of Sciences 1992, 650:13; Leceta and co-workers; Advances in Neuroimmunology 1996, 6:29).

VIP exercises its biological effects by means of membrane receptors belonging to the superfamily of seven hydrophobic domains coupled to G proteins, which transduce the information to the final effecter molecules (Laburthe M and Couvineau A; Annals of the New York Academy of Sciences 1988, 527:296). The receptors for VIP have been characterised in numerous tissues such as liver and adipose tissue, among others, and correspond to two types, the so-called VIP1 -R (Ishihara T and co-workers; Neuron 1992, 8:811) and VIP2-R (Lutz E. and co-workers; FEBS Letters 1993, 334:3). In the immune system, receptors specific to VIP have been characterised in a variety of immune cells which include human peripheral lymphocytes, human monocytes, rat and mouse lymphocytes, alveolar macrophages of rat and peritoneal macrophages of rat and mouse (Gomariz RP and co-workers; Biochemical and Biophysical Research Communications 1994, 203:1599; Delgado M and co-workers; Regulatory Peptides 1996, 62:161). VIP modulates a wide variety of immune functions such as the phagocyte function, in each one of the stages of the process, the proliferate response, the production of immunoglobins, the NK activity and the production of cytokines (De La Fuente M and co-workers; Advances in Neuroimmunology 1996, 6:75).

The adenylate cyclase hypophysary peptide activator (ACHPA) is a member of the family of peptides of the secretin/VIP/glucagons of which two molecular forms are known: ACHPA-38 and ACHPA-27, whose sequences are respectively (Ogi K and co-workers; Biochemical and Biophysical Research communication 1993, 196:1511):

ACHPA-38
His-Ser-Asp-Gly-Ile-Phe-Thr-Asp-Ser-Tyr-Ser-Arg-Tyr-Arg-Lys-Gln-Met-Ala-Val-
Lys-Lys-Tyr-Leu-Ala-Ala-Val-Leu-Gly-Lys-Arg-Tyr-Lys-Gln-Arg-Val-Lys-Asn-Lys-
NH2

ACHPA-27
His-Ser-Asp-Gly-Ile-Phe-Thr-Asp-Ser-Tyr-Ser-Arg-Tyr-Arg-Lys-Gln-Met-Ala-Val-
Lys-Lys-Tyr-Leu-Ala-Ala-Val-Leu-NH2

Both peptides are widely distributed in the central nervous system and peripheral system.

There are also cells that produce ACHPA in the lung, pancreatic B cells and intestine (Arimura A; Regulatory Peptides 1992, 37:287). In the immune system, a large abundance of ACHPA positive cells has been described in central and peripheral lymphoid organs (Gaytan F and co-workers; Cell and Tissue Research 1994, 276:233). For ACHPA, three types of receptor have been described (Shivers BD and co-workers; Endocrinology 991, 128:3055; Inagaki N and co-workers; Proceeding of the National Academy of Sciences USA 1994, 91:2679). The type-1 ACHPA (ACHPA-R-I) with equal affinity for ACHPA-38 and ACHPA-27, but which has an affinity of 300 to 1000 times less for VIP; the type-2 ACHPA receptor (ACHPA-R-II) which recognises VIP, ACHPA-38 and ACHPA-27 with the same affinity, and so is denominated the VIP-ACHPA common receptor and corresponds to the receptor of VIP VIP1-R, and the type-III ACHPA receptor (ACHPA-R-III) which corresponds to the receptor of VIP VIP2-R. Until present, there are few studies on the biological actions of ACHPA in the immune system. The effects of ACHPA are often similar to those of VIP modulating the phagocyte function and the proliferate responses.

### DESCRIPTION OF THE INVENTION

The object of this invention is to develop preparations of VIP, ACHPA and analogues as therapeutic agents in the treatment of autoimmune diseases.

The agent used in the invention inhibits the production of tumoral necrosis factor (TNF) or IL-6 or increases the production of IL-4, inhibiting the activation of Th1 cells and stimulating the activation of Th2 cells in mammals, in need thereof.

It is known that most of the effects of endotoxic shock are mediated by the activation of the immune system and the inflammatory mechanisms of the host as a response to the bacterial products. The macrophages play a very relevant role in this process as, after their activation, they produce factors such as nitric oxide, prostaglandins and cytokines which are responsible for symptoms such as fever, hypotension, disseminated microcoagulation, multiple organ failure and finally death. In this sense, high circulating levels of TNF, IL-1 and IL-6 associated with endotoxaemia have been described. In animal models these symptoms are reproduced both by the administration of bacterial endotoxins (LPS) and by injection of TNF and IL-1. Other studies have shown the diagnostic value in terms of probability of survival that circulating levels of IL-6 represent (Stoiser B and co-workers; European Journal of Clinical Investigation 1998, 28:672).

The tumoral necrosis factor (TNF) is produced by several types of cell which include monocytes and macrophages, T and B lymphocytes, neutrophils, mast cells, tumoral cells and fibroblasts. It is an important regulatory factor of other pro-inflammatory cytokines, such as IL-1β, IL-6 and IL-8. TNFα induces the expression of adhesion molecules in endothelial cells, activates the leukocytes so that they destroy the microorganisms, acts on the hepatocytes in order to increase synthesis of serum proteins which contribute to the response in acute phase and activate the coagulation system. Overproduction of this molecule leads to immunopathological diseases, autoimmunity and inflammation.

IL-6 is a multifunctional cytokine produced both by lymphocytes and by non-lymphoid cells. It regulates several aspects of the immune response, such as production of proteins that mediate the acute phase of haematopoiesis. In addition, it acts as a mediator in the inflammatory response. Its production is regulated by several factors which include TNFα, IL-1 and bacterial endotoxin (LPS).

IL-4 is a cytokine that inhibits the production of pro-inflammatory cytokines, promotes the proliferation and differentiation of activated lymphocyte B molecules and increases the expression of MHC molecules of type II and B lymphocytes. Its possible clinical use in anti-inflammatory treatment and autoimmune diseases has been highlighted.

Strategies have been tried for neutralising pro-inflammatory cytokines in the treatment of endotoxic shock but the results do not indicate that there is greater survival in the long term. A treatment that inhibits the production of TNFα and IL-6 would represent a considerable improvement in the evolution of endotoxic shock and in the probabilities of survival. The administration of VIP and ACHPA in animal models achieved these effects and our invention consists in the use of a treatment with these neuropeptides to increase the survival in endotoxic shock and revert pathological inflammatory states and autoimmune diseases.
VIP and ACHPA have anti-inflammatory effects and inhibit the production of IL-6 and TNFα in animal models of induction of endotoxic shock. As these cytokines play an important role in the development of said syndrome, VIP and ACHPA can be used to regulate their production. In addition, VIP and ACHPA modulate the capacity of the antigen presenting cells to act inducing the activation of proliferation and differentiation of lymphocytes with a pattern of cytokine secretion typical of Th2 cells and condition the immune responses "in vivo" favouring the development of response of the humoral type.

Thus an object of the present application is the use of the vasoactive intestinal peptide (VIP) or a fragment or derivative thereof for the preparation of a drug destined to the treatment of autoimmune pathologies, such as rheumatoid arthritis, multiplesclerosis, Crohn's disease and implant reaction (Graft versus host) to host in mammals.

Another object of the present application id the use of the adenylate cyclase hypophysary activator peptide (ACHPA) or a fragment or derivative thereof for the preparation of a drug destined to the treatment of autoimmune pathologies characterised by the activation of Th1 cells, such as rheumatoid arthritis, multiplesclerosis, Crohn's disease, implant reaction to host and others.

### DESCRIPTION OF THE FIGURES

Figure 1 represents the production of TNFα by murine macrophages in culture (5x10⁵ cells/ml) stimulated with 10ngr/ml of LPS in the presence or absence of 10⁻⁸M of VIP or ACHPA during the course of 24 hours.
Figure 2 represents the production of TNFα by murine macrophages in culture (5x10⁵ cells/ml) after 6 hours of culture with 10ngr/ml of LPS and to which 10⁻⁸M of VIP or ACHPA are added at different times.
Figure 3 represents the production of IL-6 by murine macrophages in culture (5x10⁵ cells/ml) stimulated with 10ngr/ml of LPS in the presence or absence of 10⁻⁸M of VIP or ACHPA during the course of 24 hours.
Figure 4 represents the production of IL-6 by murine macrophages in culture (5x10⁵ cells/ml) after 6 hours of culture with 10ngr/ml of LPS and to which 10⁻⁸M of VIP or ACHPA are added at different times.
Figure 5 presents the Northern Blot analysis for the presence of mRNA of TNFα and IL-6 in macrophages stimulated with LPS in the presence or absence of VIP or ACHPA (18S represents the corresponding rRNA as total quality control of loaded RNA).
Figure 6 represents the survival in mice injected with 400µgr of LPS and, simultaneously, or after 30 minutes, 1 or 4 hours, with 5 nmol of VIP or ACHPA.
   A. Control; B: VIP at 0h.; C: VIP at 0,5 h; D: VIP at 1 h.; E: VIP at 4 h.
Figure 7 represents the number of IL-4 secreting cells in the spleen and peritoneum detected by means of the immunabsorption assay technique in conjugated plate with enzymes (ELISPOT) in mice immunised in the conditions specified in Example 7 and which simultaneously to the second injection of antigen received 5 nmol of VIP or ACHPA or an injection of saline solution.
Figure 8 represents the quantity of anti-haemocyanine immunoglobulins of snail (anti-KLH) of the isotypes IgG2a and IgG1 detectable in serum by means of the technique conjugated immunoabsorption assays with enzymes (ELISA) in immunised mice in the conditions specified in Example 8 and with the serum samples taken two weeks after the last injection.
Figure 9 represents the number of IL-4 producing cells detected by means of the ELISPOT technique in mice that were immunised in the conditions specified in Examples 7 and 8 and which, in the second injection, received or did not receive 5 nmol of VIP along with 100µgr of IgG, anti-B7.1 or anti-B7.2.

### EMBODIMENT OF THE INVENTION

The following examples are only to illustrate the results obtained and do not limit the use of the invention which is detailed in the specified claims.

### EXAMPLE 1

### VIP and ACHPA inhibit the production of TNFα in macrophages stimulated with LPS

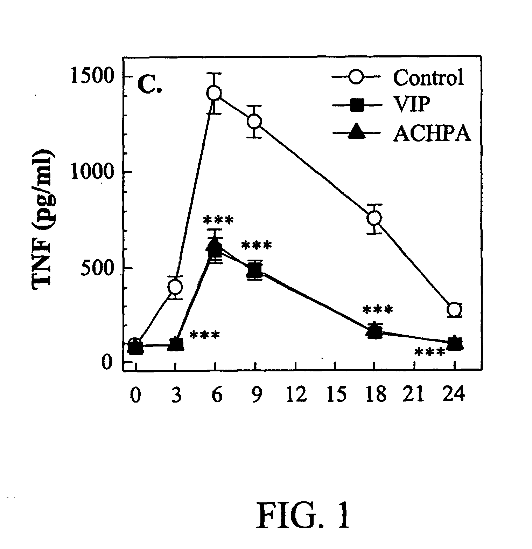

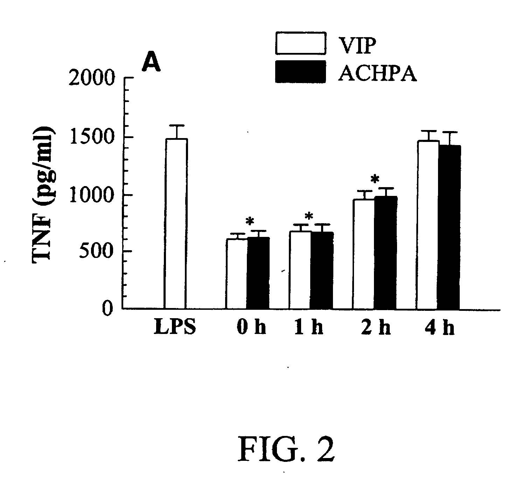

In experiments carried out "in vitro" VIP and ACHPA inhibit the production of TNFα in peritoneal murine macrophages stimulated with LPS. The greatest degree of inhibition reaches values near to 60% and occurs with doses of stimulation between 1-10 ngr./ml of LPS. The IC₅₀ value is 80 pM, both for VIP and for ACHPA and its effect was observed up until the end of the experiment (see Figure 1). The inhibitory effect is the same if both neuropeptides are added up until 1 hour after stimulating the macrophages with LPS, although it progressively reduces until disappearing if they are added after 4 hours (see Figure 2).

### EXAMPLE 2

### VIP and ACHPA reduce the circulating levels of TNFα after injection of LPS

In an experiment carried out with mice the circulating levels of TNFα 2 hours after injection of 25 µgr of LPS were approximately 4 ngr./ml. Simultaneous administration of 5 nmol of VIP or ACHPA reduced said levels by 60%.

### EXAMPLE 3

### VIP and ACHPA inhibit production of IL-6 in macrophages stimulated with LPS

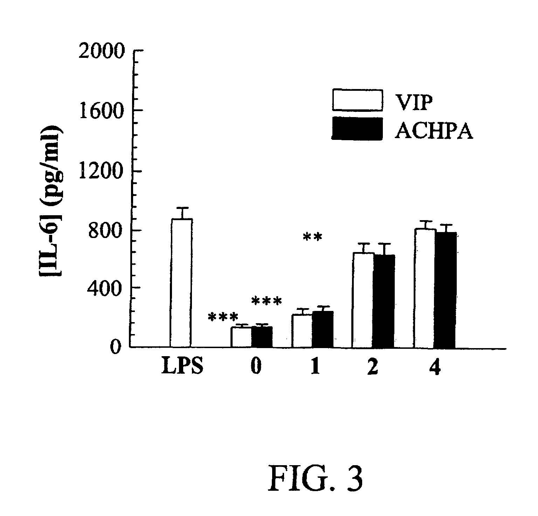

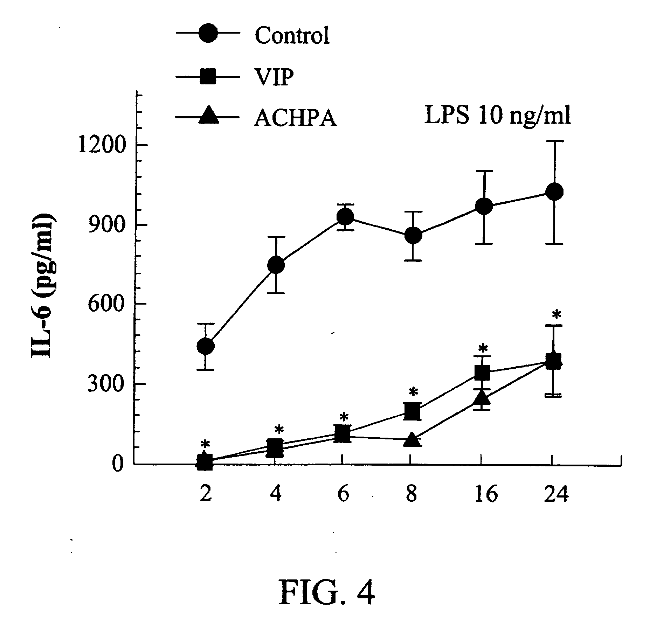

In experiments carried out " in vitro" VIP and ACHPA inhibit the production of IL-6 in peritoneal murine macrophages stimulated with LPS. Most of the inhibition reaches values near to 90% and occurs with doses of stimulation of 10 µgr./ml of LPS. The IC₅₀ is 8.6 pM, both for VIP and for ACHPA and the effect was observed up until the end of the experiment (see Figure 3). The inhibitory effect is also observed if the neuropeptides are added after stimulation with LPS, although the degree of inhibition is progressively smaller (see Figure 4).

### EXAMPLE 4

### VIP and ACHPA reduce the circulating levels of IL-6 after injection of LPS

In an experiment carried out in mice the circulating levels of IL-6 two hours after injection of 25 µgr. of LPS were approximately 1.5 ngr./ml. The simultaneous administration of 5nmol of VIP or ACHPA reduced said levels by 60% and 75%, respectively.

### EXAMPLE 5

### VIP and ACHPA regulate the production of TNFα and IL-6 at a transcriptional level

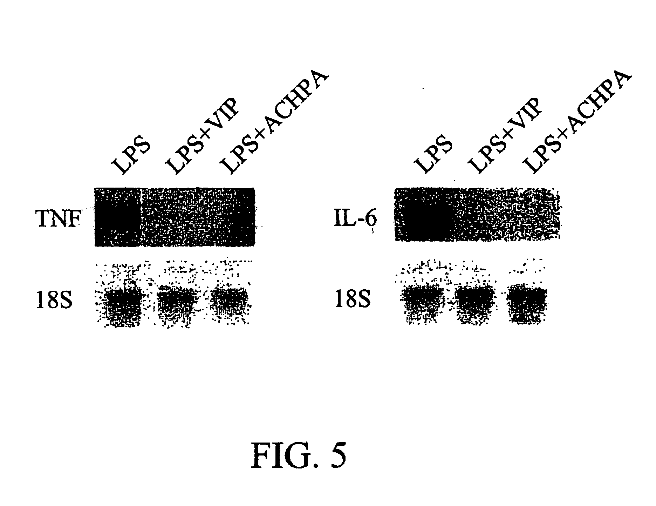

Macrophages from rat were submitted to the experimental conditions described in examples 1 and 3 and their mRNA was isolated. This mRNA was analysed using the Northern Blot technique to detect mRNA of TNFα and IL-6. Figure 5 shows the absence of transcripts for TNFα or IL-6 when the macrophages activated with LPS are also exposed to VIP or ACHPA.

### EXAMPLE 6

### VIP and ACHPA protect against the lethal effects of LPS

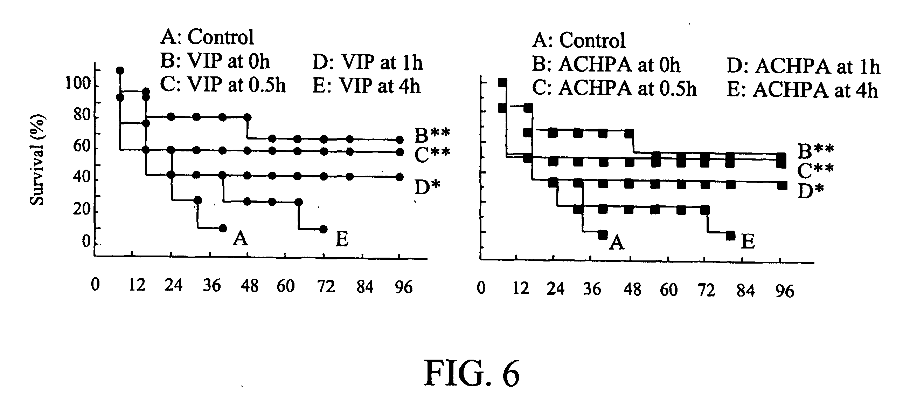

An experiment was carried out in which the long-term survival over a period of 4 days was studied for mice injected with 400µgr of LPS. The results are reflected in figure 6. The mortality in these circumstances was 100% after 36 hours. With the simultaneous administration of 5 nmol of VIP or ACHPA a survival of 60% was achieved at the end of the experiment. The administration of neuropeptides up to 1 hour after injection with LPS still gave survival rates close to 50%.

### EXAMPLE 7

### VIP and ACHPA increase the proportion of IL-4 secreting cells.

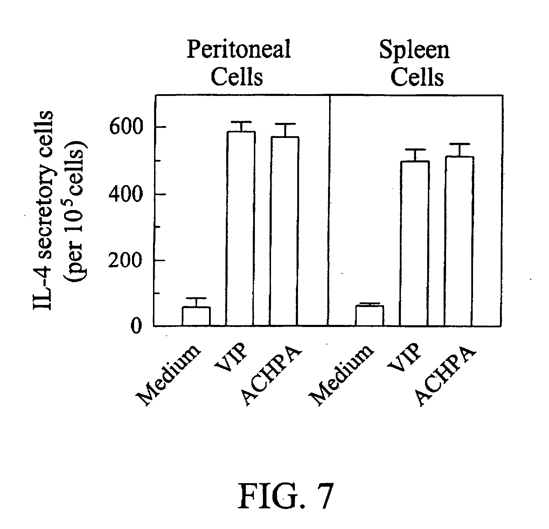

Groups of mice were immunised with 50 µgr of KLH emulsified in an adjuvant, repeating the injection with 100 µgr of KLH two weeks later and simultaneously injecting 5 nmol/mouse of VIP, ACHPA or saline solution. Two weeks after the last injection, suspensions were made of the spleen and peritoneum cells. These were cultured for 24 hours in the presence of 50 µgr/ml of KLH, after which time the number of IL-4 producing cells was determined using the ELISPOT technique. In the mice injected with VIP or ACHPA, the number of IL-4 producing cells increased in the order of 20 times compared to those that were not treated with these neuropeptides (see Figure 7).

### EXAMPLE 8

### VIP and ACHPA induce the production of antibodies of the isotype IgG1.

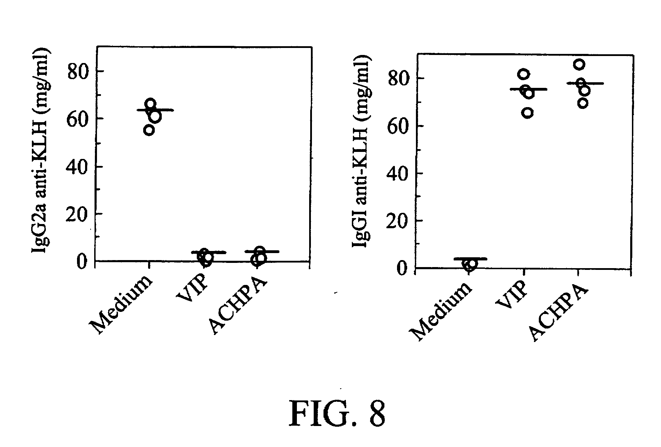

Groups of mice were immunised with 50 µgr of KLH emulsified in an adjuvant, repeating the injection with 100 µgr of KLH two weeks later and simultaneously injecting 5 nmol/mouse of VIP, ACHPA or saline solution. Two weeks after the last injection, the levels of anti-KLH and its isotype were determined using ELISA specific to the IgG1 and IgG2a isotypes. In mice injected with VIP or ACHPA the anti-KLH antibodies detectable in serum two weeks after the last immunisation are only of the IgGl isotype, while the in those that only received saline solution they were of the isotype IgG2a (see Figure 8)

### EXAMPLE 9

### The increase in the proportion of IL-4 producing cells mediated by VIP and ACHPA is related to the expression of B7.2 induced by both neuropeptides.

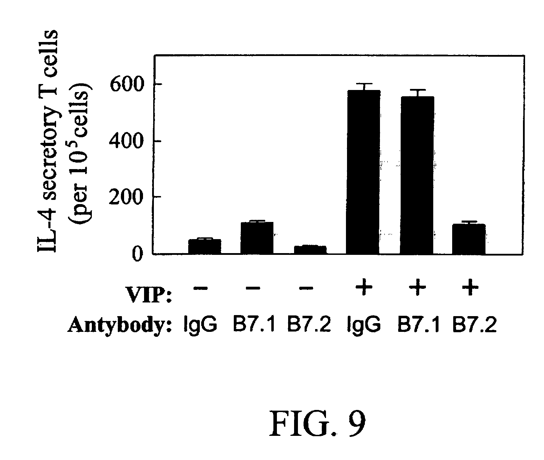

Groups of mice were immunised in the sample conditions as those for Examples 7 and 8, but in the moment of the second immunisation with KLH the mice that were simultaneously injected with VIP or ACHPA also received 100 µgr of anti-B7.1, anti-B7.2 antibody or the same quantity of IgG as control. In the mice that received anti-B7.2 antibodies simultaneously to the administration of neuropeptides the number of IL-4 producing cells was reduced to the proportion reached in animals that were not injected with neuropeptides (see Figure 9).

## Claims

1. Use of the vasoactive intestinal peptide (VIP) for the preparation of a drug destined to the treatment of autoimmune pathologies, such as rheumatoid arthritis, multiplesclerosis, Crohn's disease and implant reaction (Graft versus host) to host in mammals.

2. Use of a fragment or derivative of the vasointestinal peptide (VIP) according to claim 1.

3. Use of the adenylate cyclase hypophysary activator peptide (ACHPA) for the preparation of a drug destined to the treatment of autoimmune pathologies **characterised by** the activation of Th1 cells, such as rheumatoid arthritis, multiplesclerosis, Crohn's disease, implant reaction to host and others.A

4. Use of a fragment or derivative of the adenylate cyclase hypophysary activator peptide (ACHPA) according to claim 3.

## Patentansprüche

1. Verwendung des vasoaktiven intestinalen Peptids (VIP) zur Herstellung eines Medikaments für die Behandlung autoimmuner Erkrankungen wie rheumatische Arthritis, Multiple Sklerose, Ileitis und Reaktion des Implantats auf den Rezeptor bei Säugetieren.

2. Verwendung eines Teils oder Derivats des vasoaktiven intestinalen Peptids (VIP) gemäss Anspruch 1.

3. Verwendung des die hypophysäre Adenilatciclasa aktivierenden Peptids (ACHPA) zur Herstellung eines Medikaments für die Behandlung autoimmuner Erkrankungen wie rheumatische Arthritis, Multiple Sklerose, Ileitis und Reaktion des Implantats auf den Rezeptor oder andere, **dadurch gekennzeichnet, dass** die Th1-Zellen aktiviert werden.

4. Verwendung eines Teils oder Derivats des die hypophysäre Adenilatciclasa aktivierenden Peptids (ACHPA) gemäss Anspruch 3.

## Revendications

1. Utilisation du peptide vaso-actif intestinal (VIP) pour la préparation d'un médicament destiné au traitement de pathologies auto-immunes, telles que l'arthrite rhumatoïde, la sclérose multiple, la maladie de Crohn et la réaction de la greffe face au receveur (greffe contre receveur) chez les mammifères.

2. Utilisation d'un fragment ou dérivé du peptide vaso-actif intestinal (VIP) selon la revendication 1.

3. Utilisation du peptide activateur de l'adénilate cyclase hypophysaire (ACHPA) pour la préparation d'un médicament destiné au traitement de pathologies auto-immunes, **caractérisée par** l'activation des cellules Th1, telles que l'arthrite rhumatoïde, la sclérose multiple, la maladie de Crohn et la réaction de la greffe face au receveur et autres.

4. Utilisation d'un fragment ou dérivé du peptide activateur de l'adélinate cyclase hypophysaire (ACHPA) selon la revendication 3.
